# EUROPEAN PATENT APPLICATION

(11) **EP 2 311 508 A1**
(43) Date of publication of application: **20.04.2011**
(21) Application number: 09797967.8
(22) Date of filing: 16.07.2009
(51) Int. Cl.: A61M 1/18

(54) **BLOOD PURIFIER MANUFACTURING METHOD AND BLOOD PURIFIER**

(30) Priority: 17.07.2008 JP 2008185598
(71) Applicant: Nikkiso Company Limited, Tokyo 150-8677 (JP)
(72) Inventor: OHDAIRA, Hisahide, Kanazawa-shi Ishikawa 920-0177 (JP); CHIBA, Toshiaki, Kanazawa-shi Ishikawa 920-0177 (JP); HAYASHI, Seishu, Kanazawa-shi Ishikawa 920-0177 (JP); NAKAO, Michiharu, Kanazawa-shi Ishikawa 920-0177 (JP)
(74) Representative: Grosse, Wolfgang
(86) International application number: PCT/JP2009/062877
(87) International publication number: WO 2010/008043

(57) **Abstract**

A manufacturing method for a dry type blood purifier (1) not preloaded with water is characterized as comprising a first water-content adjustment step (a first drying step) during which average water-content value of a hollow fiber membrane is adjusted to 50 wt% or more but less than 300 wt% at a stage preceding a sterilization step using exposure to radiation, and a second water-content adjustment step (a second drying step) during which the average water-content value of the hollow fiber membrane is adjusted to less than 50 wt% after the sterilization step.

## Description

### TECHNICAL FIELD

The present invention relates to a manufacturing method of a blood purifier for use in hemodialysis therapy, hemodiafiltration therapy, or the like, and to a blood purifier which is manufactured by the manufacturing method.

### BACKGROUND ART

In blood purification therapy, a semipermeable membrane or an ultrafilter membrane which is spun in a hollow fiber shape (which is referred to as a "hollow fiber membrane") is used, and a blood purifier which is formed by loading a casing with a bundle of approximately 5000 to 10000 hollow fibers is used. By passing blood on the inner surface side of the hollow fiber membrane in the blood purifier, uremic substances are separated and removed by filtration. Further, in hemodialysis therapy, by passing blood on the inner surface side of the hollow fiber membrane and simultaneously passing a dialysis solution on the outer surface side of the hollow fiber membrane in the blood purifier to bring the blood and the dialysis solution in contact with each other via the hollow fiber membrane, the uremic substances are removed by diffusion and excess water within a body is also removed. Also, in hemodiafiltration therapy, the uremic substances and excess water within the body are removed by the properties of both the blood filtration therapy and the hemodialysis therapy, i.e. by filtration and diffusion.

The blood purifiers described above are classified into types of device preloaded with water and types of device not preloaded with water (which is referred to as a dry type). The latter type, which is not preloaded with water, is advantageous over the former type, in that the device is lightweight, easy to handle, and unlikely to be broken, even at low temperatures due to expansion caused by freezing of the water. Accordingly, these dry type blood purifiers are being preferably used.

While various types of membranes may be used as the hollow fiber membrane for use in the above-described blood purifier, a polyester polymer alloy (which will be referred to as PEPA (registered mark)) membrane formed mainly of a polyarylate resin (PAR) and a polyether sulfone resin (PES), for example, has been put into a practical use. The PEPA membrane not only has mechanical strength, heat resistance, chemical resistance, and excellent biocompatibility, but also has high adsorbing property with respect to a pyrogenic substance such as endotoxin in the dialysis solution. Consequently, there has been a great demand for development of a dry type blood purifier in which this PEPA membrane is used.

As the PEPA membrane is a hydrophobic material, it is difficult to use the PEPA membrane as it is and achieve the permeability inherent thereto. Accordingly, polyvinylpyrrolidone (PVP), which is a hydrophilic polymer, is used as a hydrophilizing agent to hydrophilize the hollow fiber membrane. At the time of actual use, in order to enable the PEPA membrane to achieve the inherent permeability, activation processing (priming) is performed, in which the blood purifier is loaded with an isotonic sodium chloride solution.

Here, during the manufacturing process of the above blood purifier, there are cases in which sterilization processing is performed by irradiating the blood purifier with radiation, for example γ rays. At the time of this sterilization processing, if the quantity of water contained in the hollow fiber membrane is low, i.e. if oxygen is present around the hollow fiber membrane, oxygen radicals are generated by irradiation with radiation. These oxygen radicals may damage polymer forming the hollow fiber membrane and hydrophilic polymer applied as a hydrophilizing agent. This leads to problems that the permeability of the hollow fiber membrane is lowered and that the polymer is eluted at the time of the activation processing. On the other hand, if the hollow fiber membrane is made to contain excess water for the purpose of addressing the above problems, air lock is produced during the activation processing. Here, the air lock refers to a state in which, at the time of using a blood purifier, air bubbles enter a portion of the hollow fiber membrane and cannot be removed. In this air lock state, the air bubbles inhibit flow of the blood, which may result in "remaining blood", in which a portion of the blood coagulates and remains within the hollow fiber membrane.

In order to address the above problems, a method has been proposed in which the sterilization processing is performed while the hollow fiber membrane is placed in a wet state having a percentage of saturated water-content or higher, by using water or an innoxious soluble substance, thereby preventing degradation of the hollow fiber membrane, for example (see Patent Document 1).

Further, as a method for suppressing degradation of the hollow fiber membrane caused by irradiation with radiation, there is a method in which the sterilization processing is performed while the hollow fiber membrane is moistened with water which is 100% or more with respect to the self weight of the hollow fiber membrane, to place the interior of the dialysis container in an inert-gas atmosphere (see Patent Document 2, for example).

Further, a hollow fiber membrane module in which the water-content of a hollow fiber membrane is optimized and which is configured such that a volume of gas to be discharged by passing a predetermined quantity range of water along the inner side of the hollow fiber membrane is a predetermined value or less, thereby suppressing generation of air lock phenomenon, is proposed (see Patent Document 3, for example).

Also, as a method for preventing the air lock, a method of hydrophilizing a hollow fiber membrane with polyhydric alcohol, polyvinylpyrrolidone, or the like, is proposed (see Patent Document 4, for example).

Moreover, a structure in which a difference in the water-content between the center portion of a hollow fiber membrane and the outer peripheral portion thereof at the time of start of drying is made to fall within a predetermined range is disclosed (see Patent Document 5, for example).

### PRIOR ART

### PATENT DOCUMENT

PATENT DOCUMENT 1: JP 55-023620 B2
PATENT DOCUMENT 2: JP 2001-170167 A
PATENT DOCUMENT 3: JP 2007-229096 A
PATENT DOCUMENT 5: JP 3815505 B2
PATENT DOCUMENT 6: JP 3992185 B2

### DISCLOSURE OF THE INVENTION

### TECHNICAL PROBLEMS

However, none of the above patent documents disclose a structure for effectively achieving both the suppression of damage to a hollow fiber membrane at the time of sterilization processing caused by radiation irradiation and the prevention of air lock at the time of the activation processing.

The present invention was proposed in view of the above problems and is aimed at providing a manufacturing method of a blood purifier in which damage to a hollow fiber membrane at the time of sterilization processing caused by radiation irradiation can be suppressed, and also generation of air lock at the time of activation processing can be prevented to effectively exhibit the permeability of the hollow fiber membrane, and is also aimed at providing a blood purifier,

### SOLUTION TO PROBLEMS

The present invention has been proposed to attain the above objects. In accordance with an aspect of the invention described in Claim 1, there is provided a manufacturing method for a blood purifier in which a hollow fiber bundle formed of a bundle of hollow fiber membranes is loaded into a casing, the manufacturing method including a first water-content adjustment step for adjusting an average value of a water-content of a hollow fiber membrane to 50 wt% or greater and less than 300 wt% at a stage preceding a sterilization step performed by irradiation with radiation, and a second water-content adjustment step for adjusting an average value of the water-content of the hollow fiber membrane to less than 50 wt% after the sterilization step.

In accordance with an aspect of the invention according to Claim 2, in the manufacturing method for a blood purifier as defined in Claim 1, a concentration of oxygen around the hollow fiber membrane during the sterilization step after the first water-content adjustment step is 5% or less.

In accordance with an aspect of the invention according to Claim 3, in the manufacturing method for a blood purifier as defined in Claim 1 or 2, a difference between a maximum value and a minimum value of water-contents of the hollow fiber membrane measured at a plurality of points in the longitudinal direction of the hollow fiber membrane during the first water-content adjustment step is 50% or less of the maximum value.

In accordance with an aspect of the invention according to Claim 4, in the manufacturing method for a blood purifier as defined in any one of Claims 1 to 3, a difference between a maximum value and a minimum value of water-contents of the hollow fiber membrane measured at a plurality of points in the longitudinal direction of the hollow fiber membrane during the second water-content adjustment step is 50% or less of the maximum value.

In accordance with an aspect of the invention according to Claim 5, in the manufacturing method for a blood purifier as defined in any one of Claims 1 to 4, during the second water-content adjustment step, a water-permeable material is used for a packaging material for packing the blood purifier to allow water contained in the hollow fiber membrane to be discharged out of the packaging material by permeation through the packaging material, thereby reducing the water-content of the hollow fiber membrane after the sterilization step.

In accordance with an aspect of the invention according to Claim 6, in the manufacturing method for a blood purifier as defined in any one of Claims 1 to 4, during the second water-content adjustment step, the blood purifier is dried by using a dryer, with the blood purifier being packed with a packaging material, to thereby reduce the water-content of the hollow fiber membrane after the sterilization step.

In accordance with an aspect of the invention according to Claim 7, in the manufacturing method for a blood purifier as defined in any one of Claims 1 to 6, a polymer alloy membrane composed of a polyarylate resin and a polyether sulfone resin is used as a primary membrane material of the hollow fiber membrane.

In accordance with an aspect of the invention according to Claim 8, in the manufacturing method for a blood purifier as defined in any one of Claims 1 to 7, during a hydrophilizing step for hydrophilizing a blood contacting portion of the hollow fiber membrane by using a hydrophilic polymer, which is performed at a stage preceding the first water-content adjustment step, polyvinylpyrrolidone is used as the hydrophilic polymer.

In accordance with an aspect of the invention according to Claim 9, in the manufacturing method for a blood purifier as defined in any one of Claims 1 to 8, during the first water-content adjustment step, drying is performed by irradiation with microwaves to thereby adjust the water-content of the hollow fiber membrane.

In accordance with an aspect of the invention according to Claim 10, there is provided a blood purifier which is manufactured by the manufacturing method according to any one of Claims 1 to 9.

### ADVANTAGEOUS EFFECTS OF INVENTION

According to the present invention, the following advantageous effects can be achieved.

Specifically, in a dry type blood purifier not preloaded with water, as the water-content of a hollow fiber membrane is optimized during the first water-content adjustment step and the second water-content adjustment step, damage to the hollow fiber membrane and the hydrophilizing agent caused by irradiation with radiation during the sterilization step can be suppressed to inhibit elution of the hollow fiber membrane and the hydrophilizing agent into blood at the time of using the blood purifier, and also generation of air lock at the time of activation processing can be suppressed to increase deaeration. As a result, it is possible to allow effective exertion of permeability inherent to the blood purifier. It is more preferable that the concentration of oxygen around the hollow fiber membrane during the sterilization step after the first water-content adjustment step is 5% or less.

### BRIEF DESCRIPTION OF THE DRAWINGS

FIG. 1 is a cross sectional view of a blood purifier which is illustrated with a middle portion in the longitudinal direction being partially cut;
FIG. 2A is a view for explaining a cross section of a hollow fiber bundle at the end portion of the casing;
FIG. 2B is a partial enlarged view of FIG. 2B; and
FIG. 3 is a flowchart for explaining steps of manufacturing a blood purifier.

### BEST MODE FOR CARRYING OUT THE INVENTION

A preferred embodiment of the present invention will be described with reference to the drawings. While in the embodiment described below, various limitations are made as preferable specific examples of the present invention, the scope of the present invention is not limited to these examples unless the following description includes specific descriptions for limiting the present invention.

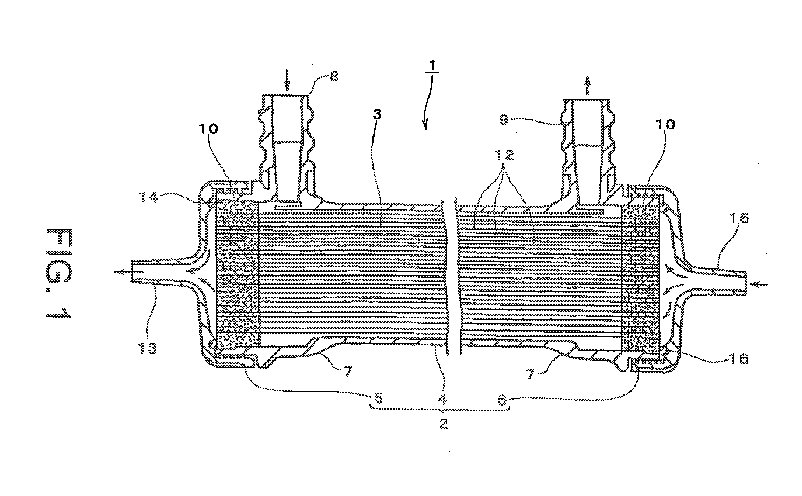

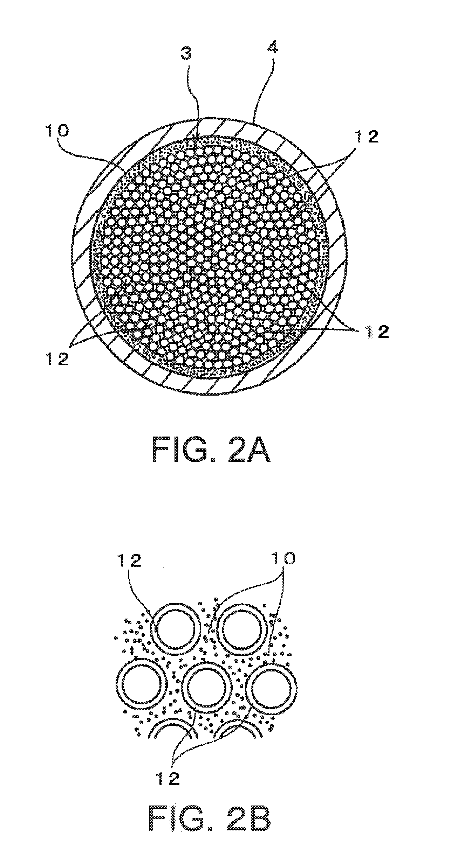

With reference to FIGs. 1 and 2A and 2B, a structure of a blood purifier 1 will be described. The blood purifier 1 which is illustrated has a structure in which a case 2 includes a hollow fiber bundle 3 therein.

The case 2 includes a cylindrical casing 4 capable of storing the hollow fiber bundle 3, a discharge side cap member 5 which is screwed onto and mounted on an opening portion at one end of this casing 4, and an infusion side cap member 6 which is screwed onto and mounted on an opening portion at the other end of this casing 4. The casing 4 is a cylindrical member having enlarged diameter portions 7 at the respective ends thereof in the longitudinal direction, and is formed of polycarbonate, for example. Further, a dialysis solution (a purification solution) infusion port 8 communicating with the internal space of the casing 4 is provided on the enlarged diameter portion 7 located at one end side in the longitudinal direction of the cylinder so as to project toward the side direction of the cylinder, and a dialysis solution discharge port 9 communicating with the internal space of the casing 4 is provided on the enlarged diameter portion 7 located at the other end side so as to project toward the side direction of the cylinder.

Seal portions 10 are provided on the respective end portions in the longitudinal direction of the cylinder in the casing 4. The seal portion 10 is a portion for fixing the end portion of the hollow fiber bundle 3 to the casing 4 by adhesion thereby sealing the casing 4, and is formed of a sealing material, for example. As the sealing material, a resin composition, such as a urethane adhesive is used. As illustrated in FIG. 2A, the sealing material fills a space between the hollow fiber bundle 3 and the casing 4 and a space among hollow fiber membranes 12 forming the hollow fiber bundle 3. With this structure, on both end surfaces of the casing 4, a plurality of hollow fiber membranes 12 having open ends are densely packed and also gaps among the hollow fiber membranes 12 are closed with the sealing material to provide a sealed state.

The seal portions 10 are provided toward the end portion sides in the longitudinal direction of the cylinder with respect to the positions of the openings of the dialysis solution infusion port 8 and the dialysis solution discharge port 8 (see FIG. 1). Accordingly, the internal space of the casing 4 is segmented into an inner side space and an outer side space of the hollow fiber membranes 12 by the hollow fiber membranes 12 and the seal portions 10. Further, the outer side space of the hollow fiber membranes 12 communicates with the outside of the casing 4 through the dialysis solution infusion port 8 and the dialysis solution discharge port 9. Also, the inner side space of the hollow fiber membrane 12 functions as a first liquid passing space for passing a liquid to be purified, such as blood, and the outer side space of the hollow fiber membrane 12 functions as a second liquid passing space for passing a purification solution such as dialysis solution.

The discharge side cap member 5 is a cap member of a substantially funnel shape including a blood discharge port 13 and is screwed onto and mounted on the other end side of the cylinder in the longitudinal direction thereof. The discharge side cap member 5 includes an 0-ring 14 which can be closely adhered to the outer peripheral portion of the outer surface of the seal portion. This 0-ring 14 adheres closely to the outer surface of the seal portion to thereby seal the boundary portion between the casing 4 and the discharge side cap member 5 in a liquid tight manner. Further, the infusion side cap member 6 is a cap member of a substantially funnel shape including a blood infusion port 15, having a structure similar to that of the discharge side cap member 5. The infusion side cap member 6 is screwed onto and mounted on the one end side of the casing 4 in the longitudinal direction thereof. In a state in which the infusion side cap member 6 is mounted, an O-ring 16 closely adheres to the outer surface of the seal portion to thereby seal the boundary portion between the casing 4 and the infusion side cap member 6 in a liquid tight manner. In the state in which the cap members 5 and 6 are mounted, the internal space of the discharge side cap member 5 and the internal space of the infusion side cap member 6, together with the inner side space of each hollow fiber membrane 12, form a blood flow passage through which blood flows,

As illustrated in FIG. 2B, the hollow fiber membrane 12 is a semipermeable membrane having a hollow fiber shape, which is very thin with a thickness of a membrane base material being approximately 5 to 50 micrometers and the inner diameter thereof being approximately 100 to 500 micrometers. Further, the seal portions 10 are formed on both end portions which are fixed with a resin composition.

The membrane base material is a semipermeable membrane of hydrophobic polymer, which is formed of a polymer alloy membrane composed of a polyarylate resin (PAR) and a polyether sulfone resin (PES) as a primary membrane material.

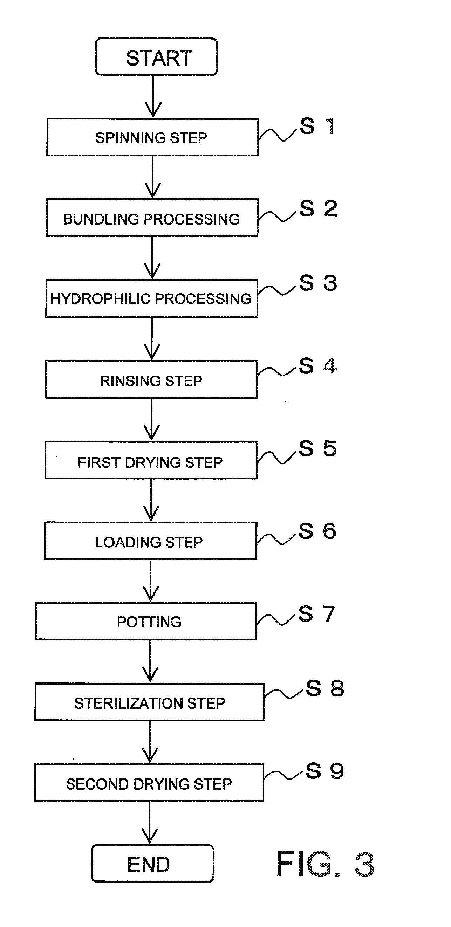

Next, with reference to a flowchart illustrated in FIG. 3, a process for manufacturing the blood purifier as described above will be described.

In this manufacturing process, spinning of a hollow fiber is first performed (step S1).

In this spinning step, preparation of a membrane-forming stock solution is initially performed. This membrane-forming stock solution for spinning a hydrophobic membrane base material is prepared by dissolving a polyarylate resin (A) and a polyether sulfone resin (B) in an organic solvent such that the weight ratio of the mixture (A/B) of the polyarylate resin (A) and the polyether sulfone resin (B) falls within the range of 0.1 to 10 and also the total quantity (A+B) of the two resins is between 10 wt% and 25 wt%. In the present embodiment, the weight ratio of the mixture is set to 1 (A:B=1:1).

By discharging the membrane-forming stock solution prepared as described above along with a core liquid into a coagulating liquid by using a double-tube spinneret, a membrane base material in a hollow fiber shape including polyester polymer alloy as a membrane raw material, i.e. the hollow fiber membrane 12, can be obtained. Here, as the core liquid and the coagulating liquid, N-methyl pyrrolidone, for example, can be used.

The hollow fiber membrane 12 includes a dense layer (inner dense layer) formed on the inner surface side thereof, and also includes a porous layer having a great number of pores formed so as to cover the outer side of this dense layer. In the case of a PEPA membrane, an additional dense layer (an outer dense layer) is further formed on the outer side of the porous layer. The inner dense layer is a portion which defines the permeability, i.e. material selection permeation ability and a permeation rate in this hollow fiber membrane (membrane base material), and, in the present embodiment, has pores having a average pore size of 2 to 80 nm formed therein. Further, the porous layer functions as a supporting layer which supports the inner dense layer and the outer dense layer to thereby maintain the strength of the membrane, and includes pores having a pore size in the range of 2 to 10 µm (2,000 to 10,000 nm), which is rougher than the pore size in each dense layer. The outer dense layer is specific to the PEPA membrane, and has a pore size in the range between 5 nm and 100 nm. The pore size in each layer can be adjusted by changing the weight ratio of the mixture of the polyarylate resin and the polyether sulfone resin when forming the PEPA membrane. It is preferable that the pore size is in the range of 5 nm to 50 nm in the inner dense layer and in the range of 10 nm to 70 nm in the outer dense layer, and that the pore size in each dense layer is smaller than that in the porous layer. By defining the pore size of the inner dense layer and the pore size of the outer dense layer to predetermined sizes which are smaller than that in the porous layer as described above, it is possible not only to inhibit pyrogenic substances due to adsorption thereof caused by the hydrophobic interaction of the pyrogenic substances with the hydrophobic PEPA membrane which is the hollow fiber membrane 12, but also to prevent entering of the pyrogenic substances due to the exclusion effects by the pore size of the membrane.

Subsequently, bundling processing of the hollow fiber membranes which are spun as described above is performed (step S2). In this bundling processing, approximately 3000 to 15000 hollow fiber membranes 12 are bundled as each unit, and are further cut to a predetermined length to thereby form a hollow fiber bundle 3. The hollow fiber bundle 3 is adjusted to have an outer diameter corresponding to the inner diameter of the casing 4. In the present embodiment, approximately 10000 hollow fiber membranes are bundled to form the hollow fiber bundle 3.

Once the hollow fiber bundle 3 is obtained, hydrophilic processing (hydrophilic polymer adhering and retaining processing) is performed (step 3). While this hydrophilic processing can be performed in a state in which the hollow fiber bundle 3 is mounted on the casing 4, in the present embodiment, an example in which the hydrophilic processing is performed prior to mounting of the hollow fiber bundle 3 onto the casing 4 will be described.

Here, as the hydrophilic polymer (a hydrophilizing agent) for use in the hydrophilic processing, polyvinylpyrrolidone (PVP) is used. In this hydrophilic processing, 0.5% solution of the hydrophilizing agent is caused to flow along the blood contacting side on the inner side of the hollow fiber membrane, thereby hydrophilizing the hollow fiber membrane. When a hydrophilic polymer having a high molecular weight (70000 or more) is used, as the hydrophilizinig agent does not permeate through the hollow fiber membrane 12, during the hydrophilic processing, only the blood contacting surface of the hollow fiber membrane is hydrophilized and the outer side surface of the hollow fiber, which the hydrophilizing agent does not reach, remains hydrophobic. Accordingly, adsorption of the pyrogenic substances due to the hydrophobic interaction can be expected, so that the greater ability to inhibit the pyrogenic substances can be preferably achieved. However, a problem concerning coagulation of blood, such as remaining blood, is likely to arise when only a hydrophilizing agent having a high molecular weight is used, because the hydrophilizing agent does not reach the thickness direction of the hollow fiber membrane and therefore the thickness direction of the hollow fiber membrane is not hydrophilized. Therefore, in some cases, it is more desirable to use a hydrophilizinig agent having a low molecular weight (less than 70000) simultaneously with a hydrophilizinig agent having a high molecular weight. It could be confirmed that while the ability to inhibit the pyrogenic substances due to the hydrophobic interaction is lowered in the case of using a hydrophilizinig agent having a low molecular weight, when the outer dense layer has a fixed pore size, the ability to inhibit the pyrogenic substances is sufficient and the hydrophilizinig agent having a low molecular weight can be preferably used.

When the membrane base material of the hollow fiber membrane is formed of a polyether sulfone resin, PVP, and N-methyl pyrrolidone, the hydrophilic processing can be omitted.

termination of the hydrophilic processing, a rinsing step is subsequently performed (step S4). During this rinsing step, an excess hydrophilizing agent is removed from the hollow fiber membrane 12 of the hollow fiber bundle 3 for which the hydrophilic processing has been completed. More specifically, purified water for rinsing (rinsing water), in place of the aqueous solution of the hydrophilizing agent used in the hydrophilic processing described above, is caused to pass along the blood contacting portion on the inner side of the hollow fiber. With this rinsing step, in the hydrophilizing agent adhered to the blood contacting portion, an excess hydrophilizing agent which is adsorbed with an adsorptive force that is lower than the predetermined adsorptive force, is removed by rinsing.

Subsequently, a first drying step for drying the hollow fiber bundle 3 is performed (step S5). This first drying step corresponds to a first water-content adjustment step for adjusting the water-content of the hollow fiber membrane at a stage preceding to a sterilization step. While a drying method in which the hollow fiber bundle 3 is irradiated with microwaves and a drying method in which hot air is caused to flow through the hollow fiber bundle may be used as the drying method, in the first drying step of the present embodiment, the former method in which microwaves are used is adopted for drying. At this time, former-half drying processing with irradiation of microwaves for a predetermined time period is performed with the hollow fiber bundle 3 being in an upright position, that is, in the position in which the longitudinal direction of the hollow fiber bundle 3 is made to correspond to the vertical direction. Thereafter, with the hollow fiber bundle 3 being turned upside down, latter-half drying processing is performed by irradiating the hollow fiber bundle 3 with microwaves for a time period which is the same as the time period for the former-half drying. By performing the former-half drying processing and the latter-half drying processing for the same time period in the respective drying steps, with the upright position being turned upside down as described above, it is possible to make the water-content uniform in the longitudinal direction of the hollow fiber membrane as quickly as possible. Specifically, it is desirable that a difference between the maximum measured value and the minimum measured value of the water-contents, measured at a plurality of points on the hollow fiber membrane set at equal intervals in the longitudinal direction, is set to be within 50% or less of the maximum value.

The processing conditions in this first drying step (parameters of microwaves, or the temperature of hot air, the drying time, or the like) are related to the water-content in the hollow fiber membrane 12 (the ratio of the moisture weight with respect to the weight of the hollow fiber membrane) after drying. This water-content of the hollow fiber membrane 12 is important in view of suppression of damage to the hollow fiber membrane or the hydrophilic polymer caused by irradiation with radiation at the time of sterilization processing which will be described below. Accordingly, during the first drying step, the processing conditions are adjusted such that the average of the water-contents of the hollow fiber membrane 12 measured at a plurality of points in the longitudinal direction thereof falls within 50 wt% or greater and less than 300 wt% with respect to the weight of the hollow fiber membrane 12. With the adjustment of the water-content in this manner, the contacting area between the hollow fiber membrane and the ambient atmosphere is reduced. Here, the average water-content should be less than 300 wt%, because, with the water-content 300 wt% or greater, no increase in the damage suppression effect of the hollow fiber member 12 or the like can be expected. More specifically, as the maximum quantity of water which the hollow fiber membrane 12 can contain is less than 300 wt%, with the quantity of water greater than that, the hollow fiber membrane 12 becomes supersaturated. Therefore, if the water-content is 300 wt% or greater, water which cannot be contained in the hollow fiber membrane 12 forms a water droplet, which then adheres to the surface of the hollow fiber membrane.

After completion of the first drying step, the hollow fiber bundle 3 is loaded into the casing 4 (loading step; step S6). During this loading step, the hollow fiber bundle 3 after drying is loaded into the casing 4 with the blood infusion port 15 and the blood discharge port 13 being detached therefrom.
In a state in which the hollow fiber bundle 3 is loaded into the casing 4, the both end portions of the hollow fiber bundle 3 extend off the edges of the casing 4.

Then, potting is performed (step S7). During this potting processing, the opening of the casing 4 is sealed with a polyurethane resin serving as a sealing member to form the seal portion 10, and also the portion of the hollow fiber membrane 3 extending off the casing 4 is cut to be coplanar with the opening of the casing 4. With the above processing, a module in which the hollow fiber bundle 3 is loaded into the casing 4 is formed. According to the present embodiment, a module having a membrane area of 1.5 m² is formed.

As illustrated in FIG. 2A, the cross section of the hollow fiber bundle 3 is in a state in which a great number of hollow fiber membranes 12 having an open end portion are densely packed, and is also in a bound state in which the urethane resin serving as the seal portion 10 closes the gaps between the hollow fiber membranes 12 while securing liquid-tightness, Further, the seal portions 10 do not close the infusion port and the discharge port 9 for the dialysis solution. Consequently, in this module, the flow passage of blood (the inner surface side of the hollow fiber membrane 12) and the flow passage of the dialysis solution (the outer surface side of the hollow fiber membrane 12) are separated from each other by the hollow fiber membrane 12.

Then, the sterilization step is performed in a state in which the blood purifier 1 which is obtained as described above is packed by a polyurethane packaging material (a sterilization bag), for example, under the nitrogen atmosphere so that the concentration of oxygen around the hollow fiber membrane is adjusted to be 5% or less. While sterilization methods may include EOG (ethylene oxide gas) sterilization, steam sterilization, radiation sterilization, and so on, with the EOG sterilization, there is a problem of remaining gas after sterilization, and the steam sterilization suffers from significant damage to the module due to high temperatures. Accordingly, in the present embodiment, sterilization with radiation, more specifically, sterilization with the use of γ-rays irradiation or electron-beam irradiation is performed. While in this sterilization step, there arises a problem of damage to the hollow fiber membrane 12 and the hydrophilizing agent due to irradiation with radiation, because in the blood purifier 1 according to the present embodiment, the water-content of the hollow fiber membrane 12 is optimized during the first drying step as described above and the concentration of oxygen around the hollow fiber membrane during the sterilization step is adjusted to be 5% or less, it is possible to suppress oxygen radicals generated by irradiation with radiation to thereby suppress the damage to the hollow fiber membrane 12 and the hydrophilizing agent. As a result, it is further possible to prevent the hollow fiber membrane 12 or the hydrophilizing agent from eluting into blood at the time of using the blood purifier 1 to thereby suppress the reduction in the permeability.

Next, a second drying step for drying the blood purifier for which the sterilization step has been completed is performed (step S9). This second drying step corresponds to a second water-content adjustment step for adjusting the water-content of the hollow fiber membrane at a stage following the sterilization step. During this second drying step, drying is performed by heating the blood purifier 1 using a hot air dryer (which is one type of drying means in the present invention) in a state in which the blood purifier 1 is packed with the packaging material. The water-content of the hollow fiber membrane 12 after this second drying step is important from a view point of achieving excellent deaeration at the time of the activation processing. Accordingly, in the second drying process, the processing conditions (the temperature of hot air, the drying time, or the like) are adjusted such that the average value of the water-contents of the hollow fiber membrane 12 measured at a plurality of points in the longitudinal direction thereof falls within a range of less than 50 wt% with respect to the weight of the hollow fiber membrane 12. Further, in this second drying step, it is desirable to adjust the processing conditions such that a difference between the maximum measured value and the minimum measure value of the water-contents measured at a plurality of points on the hollow fiber membrane set at equal intervals in the longitudinal direction is set within 50% or less of the maximum value.

As described above, concerning the above-described dry type blood purifier 1 not preloaded with water, as the water-content of the hollow fiber membrane 12 is optimized by the first drying step and the second drying step, it is possible to suppress the damage to the hollow fiber membrane 12 and the hydrophilizing agent caused by the irradiation with radiation during the sterilization step to thereby prevent elution of the hollow fiber membrane 12 and the hydrophilizing agent into the blood at the time of the use of the blood purifier 1, and also to suppress generation of air lock during the activation processing to thereby further enhance deaeration. As a result, the permeability inherent to the blood purifier 1 can be exerted more effectively.

In order to confirm the effectiveness of the present invention, comparative experiments were performed under the following conditions. The results of the experiments are indicated in the following Tables 1 and 2. In these tables, Examples 1 to 5 show results of the experiments concerning a blood purifier 1 in which each of the conditions of the water-content of the hollow fiber membrane 12 prior to the sterilization (after the first drying step) and the water-content of the hollow fiber membrane 12 prior to use (after the second drying step) are set within the predetermined range as described above, and Comparative Examples 1 to 3 show results of the experiments concerning a blood purifier 1 in which at least any one of the conditions is set out of the predetermined range.

**[Table 1]**

| | Prior To Sterilization (after first drying step) | | | | | | |
|---|---|---|---|---|---|---|---|
| | Water-content (wt%) | | | | | Average water- content (wt%) | Difference in water-contents with respect to maximum value (%) |
| Example 1 | 71 | 71 | 67 | 59 | 48 | 63 | 32 |
| Example 2 | 66 | 65 | 56 | 62 | 50 | 60 | 24 |
| Example 3 | 66 | 65 | 56 | 62 | 50 | 60 | 24 |
| Example 4 | 291 | 285 | 287 | 285 | 284 | 286 | 2 |
| Example 5 | 71 | 72 | 68 | 61 | 54 | 65 | 25 |
| Comparative Example 1 | 105 | 102 | 98 | 95 | 87 | 97 | 17 |
| Comparative Example 2 | 105 | 99 | 95 | 82 | 50 | 86 | 52 |
| Comparative Example 3 | 45 | 44 | 40 | 38 | 32 | 40 | 29 |

**[Table 2]**

| | Prior To Use (after second drying step) | | | | | | | Air Lock | Eluting material test |
|---|---|---|---|---|---|---|---|---|---|
| | Water-content (wt%) | | | | | Average water- content (wt%) | Difference in water-contents with respect to maximum value (%) | | |
| Example 1 | 49 | 48 | 46 | 44 | 40 | 45 | 18 | No | Acceptable |
| Example 2 | 46 | 33 | 32 | 30 | 28 | 34 | 39 | No | Acceptable |
| Example 3 | 48 | 48 | 45 | 38 | 34 | 43 | 29 | No | Acceptable |
| Example 4 | 42 | 39 | 40 | 37 | 44 | 40 | 16 | No | Acceptable |
| Example 5 | 42 | 30 | 30 | 28 | 25 | 31 | 40 | No | Acceptable |
| Comparative Example 1 | 64 | 60 | 57 | 51 | 48 | 56 | 25 | Yes | Acceptable |
| Comparative Example 2 | 64 | 62 | 57 | 30 | 27 | 48 | 58 | No | Unacceptable |
| Comparative Example 3 | 15 | 12 | 10 | 9 | 8 | 11 | 47 | No | Unacceptable |

Example 1 shows a result of an experiment concerning a blood purifier 1 (with a membrane area of 1.5 m²) manufactured according to the procedure as described in the above embodiment. Concerning the membrane-forming stock solution, U polymer (registered mark), manufactured by UNITIKA LTD, was used as a polyarylate resin, and Sumika Excel PES (registered mark), manufactured by Sumitomo Chemical Co., Ltd, was used for a polyether sulfone rein, respectively, and the ratio of weight of the mixture thereof was set to 1:1. Further, as the hydrophilic polymer (hydrophilizing agent), a 0.5% mixture solution of Luvitec K90PH (registered mark) and Luvitec K30PH (registered mark) manufactured by BASF Japan ltd., (K90PH:K30PH=3:2) was used. In this Example 1, the water-contents were measured at five points which are equally spaced ', from each other in the longitudinal direction of the hollow fiber membrane 12 after the first drying step and prior to the sterilization step. The measured water-contents were 71 wt%, 71 wt%, 67 wt%, 59 wt%, and 48 wt%, respectively, and the average water-content was 63 wt%. Further, the difference between the maximum value and the minimum value was 32% of the maximum value. During the second drying step, the blood purifier 1 which was packed by the above packaging material was dried for 72 hours while setting the hot air temperature of the hot air dryer to 50°C, to thereby decrease the water-content of the hollow fiber membrane 12. Then, concerning the hollow fiber membrane 12 after being dried, the water-contents measured at five points which are equally spaced from each other in the longitudinal direction of the hollow fiber membrane 12 were 49 wt%, 48 wt%, 46 wt%, 44 wt%, and 40 wt%, respectively, and the average water-content was 45 wt%. Also, the difference between the maximum value and the minimum value was 18% of the maximum value.

Example 2 shows a result of an experiment concerning a blood purifier 1 which was manufactured by first performing the loading step (S6) and the potting processing (S7) with regard to a hollow fiber bundle 3 obtained through the spinning step (S1) and the bundling processing (S2) described in the above embodiment, and thereafter sequentially performing the hydrophilic processing (S3), the rinsing step (S4), the first drying step (S5), the sterilization step (S8), and the second drying step (S9) in this order. During the first drying step, drying was performed with the blood purifier 1 standing upright and the position of the blood purifier 1 being turned upside down for the former half processing and the latter half processing, while reducing the pressure within the module by a vacuum pump through the port 8 for infusion of dialysis solution. Further, during the second drying step, in a manner similar to Example 1 described above, the blood purifier 1 packed by a packaging material was dried for 72 hours, with the temperature of hot air of the hot air dryer being set to 50°C, thereby reducing the water-content of the hollow fiber membrane 12. In this Example 2, concerning the hollow fiber membrane 12 after the first drying step and prior to the sterilization, the water-contents measured at five points which are equally spaced from each other in the longitudinal direction of the hollow fiber membrane 12 were 66 wt%, 65 wt%, 56 wt%, 62 wt%, and 50 wt%, respectively, and the average water-content was 60 wt%. Also, the difference between the maximum value and the minimum value was 24% of the maximum value. Concerning the hollow fiber membrane 12 after the second drying step, the water-contents measured at five points which are equally spaced from each other in the longitudinal direction of the hollow fiber membrane 12 were 46 wt%, 33 wt%, 32 wt%, 30 wt%, and 28 wt%, respectively, and the average water-content was 34 wt%. Also, the difference between the maximum value and the minimum value was 39% of the maximum value.

The procedure of Example 3 is the same as that of Example 2 described above up to the first drying step. Concerning the processing in the sterilization step and the subsequent steps, Example 3 differs from Example 2 in that a second packaging material partially including Japanese paper which is water-permeable to thereby increase water permeability in that portion was used. Further, by achieving double packing by packing the blood purifier 1 packed with the second packaging material described above with a third packaging material with low water permeability until the completion of the sterilization step, a reduction in the water-content was prevented until the termination of the sterilization step. In the second drying step after the sterilization step, the third packaging material was removed to leave the blood purifier 1 packed with only the second packaging material. In this state, the blood purifier 1 was left unattended for 7 days at room temperature to allow the water contained in the hollow fiber membrane 12 to permeate through a portion of the packaging material and be discharged out of the packaging material, thereby decreasing the water-content of the hollow fiber membrane 12. In the Example 3, the water-contents at the five points on the hollow fiber membrane 12, the average value thereof, and the difference between the maximum value and the minimum value, prior to the sterilization step, are the same as those in Example 2 described above. Further, the water-contents measured at five points which are equally spaced from each other in the longitudinal direction of the hollow fiber membrane 12 after the second drying step were 48 wt%, 48 wt%, 45 wt%, 38 wt%, and 34 wt%, respectively, and the average water-content was 43 wt%. Also, the difference between the maximum value and the minimum value was 29% of the maximum value.

The procedure of Example 4 is the same as that of Example 2 described above up to the rinsing step, and differs from that of Example 2 in that, during the first drying step, excess water is removed by air blowing. In this Example 4, the water-contents measured at five points which are equally spaced from each other in the longitudinal direction of the hollow fiber membrane 12 after the first drying step and prior to the sterilization step were 291 wt%, 285 wt%, 287 wt%, 285 wt%, and 284 wt%, respectively, and the average water-content was 286 wt%. Also, the difference between the maximum value and the minimum value was 2% of the maximum value. Further, similar to Example 3, double packing was performed by packing the blood purifier 1 packed with the second packaging material with the third packaging material having low water permeability until the completion of the sterilization step. In the second drying step after the sterilization step, the third packaging material was removed to leave the blood purifier 1 packed with only the second packaging material. In this state, the blood purifier 1 was dried for 720 hours with the hot air temperature of the hot air dryer being set to 50°C. The water-contents measured at five points which are equally spaced from each other in the longitudinal direction of the hollow fiber membrane 12 after the drying were 42 wt%, 39 wt%, 40 wt%, 37 wt%, and 44 wt%, respectively, and the average water-content was 40 wt%. Also, the difference between the maximum value and the minimum value was 16% of the maximum value.

In Example 5, during the spinning processing, preparation of a polymer stock solution was performed by using N-methyl pyrrolidone, with the weight ratio of the mixture (A/B) of a polyether sulfone resin (A) and a polyvinylpyrrolidone (B) being set to 5. The subsequent processing up to the second drying step was the same as that in the Example 2 described above. In this Example 5, the water-contents measured at five points which are equally spaced from each other in the longitudinal direction of the hollow fiber membrane 12 after the first drying step and prior to the sterilization step were 71 wt%, 72 wt%, 68 wt%, 61 wt%, and 54 wt%, respectively, and the average water-content was 65 wt%. Also, the difference between the maximum value and the minimum value was 25% of the maximum value. Further, the water-contents measured at five points which are equally spaced from each other in the longitudinal direction of the hollow fiber membrane 12 after the second drying step were 42 wt%, 30 wt%, 30 wt%, 28 wt%, and 25 wt%, respectively, and the average water-content was 31 wt%. Also, the difference between the maximum value and the minimum value was 40% of the maximum value.

Further, it was confirmed that in each of Examples 1 to 5 described above, the concentration of oxygen within the packaging material after the first drying step and prior to the sterilization step was 5% or less.

The procedure of Comparative Example 1 is the same as that of Example 2 described above, except for the first drying step. In Comparative Example 1, among the processing conditions in the first drying step, the drying time was shorter than that in Example 2. In this Comparative Example 3, the water-contents measured at five points which are equally spaced from each other in the longitudinal direction of the hollow fiber membrane 12 after the first drying step and prior to the sterilization step were 105 wt%, 102 wt%, 98 wt%, 95 wt%, and 87 wt%, respectively, and the average water-content was 97 wt%. Also, the difference between the maximum value and the minimum value was 17% of the maximum value. Further, the water-contents measured at five points which are equally spaced from each other in the longitudinal direction of the hollow fiber membrane 12 after the second drying step were 64 wt%, 60 wt%, 57 wt%, 51 wt%, and 48 wt%, respectively, and the average water-content was 56 wt%. Also, the difference between the maximum value and the minimum value was 25% of the maximum value.

The procedure of Comparative Example 2 is the same as that of Example 2 described above except the first drying step. In Comparative Example 2, among the processing conditions in the first drying step, the drying time was shorter than that in Example 2, and also drying was performed without turning the blood purifier 1 upside down for the former half and the latter half of the drying processing. In this Comparative Example 2, the water-contents measured at five points which are equally spaced from each other in the longitudinal direction of the hollow fiber membrane 12 after the first drying step and prior to the sterilization step were 105 wt%, 99 wt%, 95 wt%, 82 wt%, and 50 wt%, respectively, and the average water-content was 86 wt%. Also, the difference between the maximum value and the minimum value was 52% of the maximum value. Further, the water-contents measured at five points which are equally spaced from each other in the longitudinal direction of the hollow fiber membrane 12 after the second drying step were 64 wt%, 62 wt%, 57 wt%, 30 wt%, and 27 wt%, respectively, and the average water-content was 48 wt%. Also, the difference between the maximum value and the minimum value was 58% of the maximum value.

The procedure of Comparative Example 3 is the same as that of Example 2 described above except for the first drying step. In Comparative Example 3, among the processing conditions in the first drying step, the drying time was longer than that in Example 2. In this Comparative Example 1, the water-contents measured at five points which are equally spaced from each other in the longitudinal direction of the hollow fiber membrane 12 after the first drying step and prior to the sterilization step were 45 wt%, 44 wt%, 40 wt%, 38 wt%, and 32 wt%, respectively, and the average water-content was 40 wt%, Also, the difference between the maximum value and the minimum value was 29% of the maximum value. Further, the water-contents measured at five points which are equally spaced from each other in the longitudinal direction of the hollow fiber membrane 12 after the second drying step were 15 wt%, 12 wt%, 10 wt%, 9 wt%, and 8 wt%, respectively, and the average water-content was 11 wt%. Also, the difference between the maximum value and the minimum value was 47% of the maximum value.

The activation processing was then performed with respect to the blood purifier 1 in each of Examples 1 to 5 and Comparative Examples 1 to 3, and generation of air lock and eluting materials were measured. Here, the measurement and acceptable/unacceptable determination concerning the eluting materials were performed in accordance with the following procedure.

First, 1.5g of the hollow fiber membrane 12 which was cut to about 2 cm was put in a beaker of 300 mL. After adding water which was boiled and then cooled, the beaker was covered with aluminum foil. After heating and shaking for one hour in the constant temperature shaking tank maintained at 70°C, the beaker was cooled with water to obtain a test solution. Similarly, water which was boiled and cooled was put in a beaker of 300 mL to obtain a blank test solution for comparison. With respect to the test solution, the absorbance with regard to ultraviolet rays having a wavelength of 220 nm to 350 nm was measured, while performing the measurement concerning the blank test solution for comparison. Then, concerning the test solution, a result with the absorbance of 0.1 or greater was determined to be acceptable and a result with the absorbance less that 0.1 was determined to be unacceptable.

Concerning each of Examples 1 to 5, in which all the conditions including the water-content or the like are within the predetermined range, generation of air lock was not identified, and the measurement result concerning the eluting materials was also determined to be acceptable. In Comparative Example 1, in which the average value of the water-content after the second drying step was 56%, which is slightly above the predetermined range (which is less than 50 wt%), while the measurement result concerning the eluting materials was determined to be acceptable, generation of air lock was identified during the activation processing. In Comparative Example 2, in which the differences between the maximum value and the minimum value of the water-content after the first drying step and after the second drying step were 52% and 58%, respectively, which are slightly above the predetermined range (which is 50 wt% or less of the maximum value), while generation of air lock was not identified during the activation processing, the measurement result concerning the eluting materials was determined to be unacceptable. In Comparative Example 3, in which the average value of the water-content after the first drying step was 40%, which is slightly below the predetermined range (which is 50 wt% or greater and less than 300 wt%), while generation of air lock was not identified during the activation processing, the measurement result concerning the eluting materials was determined to be unacceptable.

While the present invention has been described in detail, the scope of the present invention is not limited to the examples described above.

Further, all the contents described in the Detailed Description of the Invention, the claims, drawings, and abstract in the specification disclosed in Japanese Patent Application 2008-185598 filed on July 17, 2008, are incorporated in the present application.

### INDUSTRIAL APPLICABILITY

The present invention is preferably applicable to blood purification.

### REFERENCE NUMERALS LIST

1 blood purifier, 2 case, 3 hollow fiber bundle, 4 casing, 5 discharge side cap member, 6 infusion side cap member, 7 enlarged diameter portion, 8 dialysis solution infusing port, 9 dialysis solution discharge port, 10 seal portion, 12 hollow fiber membrane, 13 blood discharge port, 14 O ring, 15 blood infusion port.

## Claims

1. A manufacturing method for a blood purifier in which a hollow fiber bundle formed of a bundle of hollow fiber membranes is loaded into a casing, the manufacturing method comprising:
a first water-content adjustment step for adjusting an average value of a water-content of a hollow fiber membrane to 50 wt% or greater and less than 300 wt% at a stage preceding a sterilization step performed by irradiation with radiation; and
a second water-content adjustment step for adjusting an average value of the water-content of the hollow fiber membrane to less than 50 wt% after the sterilization step.

2. The manufacturing method for a blood purifier according to Claim 1, wherein
a concentration of oxygen around the hollow fiber membrane during the sterilization step after the first water-content adjustment step is 5% or less.

3. The manufacturing method for a blood purifier according to Claim 1 or 2, wherein
a difference between a maximum value and a minimum value of water-contents of the hollow fiber membrane measured at a plurality of points in the longitudinal direction of the hollow fiber membrane during the first water-content adjustment step is 50% or less of the maximum value.

4. The manufacturing method for a blood purifier according to any one of Claims 1 to 3, wherein
a difference between a maximum value and a minimum value of water-contents of the hollow fiber membrane measured at a plurality of points in the longitudinal direction of the hollow fiber membrane during the second water-content adjustment step is 50% or less of the maximum value.

5. The manufacturing method for a blood purifier according to any one of Claims 1 to 4, wherein
during the second water-content adjustment step, a water-permeable material is used for a packaging material for packing the blood purifier to allow water contained in the hollow fiber membrane to be discharged out of the packaging material by permeation through the packaging material, thereby reducing the water-content of the hollow fiber membrane after the sterilization step.

6. The manufacturing method for a blood purifier according to any one of Claims 1 to 4, wherein
during the second water-content adjustment step, the blood purifier is dried by using a dryer, with the blood purifier being packed with a packaging material, to thereby reduce the water-content of the hollow fiber membrane after the sterilization step.

7. The manufacturing method for a blood purifier according to any one of Claims 1 to 6, wherein
a polymer alloy membrane composed of a polyarylate resin and a polyether sulfone resin is used as a primary membrane material of the hollow fiber membrane.

8. The manufacturing method for a blood purifier according to any one of Claims 1 to 7, wherein
during a hydrophilizing step for hydrophilizing a blood contacting portion of the hollow fiber membrane by using a hydrophilic polymer, which is performed at a stage preceding the first water-content adjustment step, polyvinylpyrrolidone is used as the hydrophilic polymer.

9. The manufacturing method for a blood purifier according to any one of Claims 1 to 8, wherein
during the first water-content adjustment step, drying is performed by irradiation with microwaves to thereby adjust the water-content of the hollow fiber membrane.

10. A blood purifier which is manufactured by the manufacturing method according to any one of Claims to 9.
